# EUROPEAN PATENT SPECIFICATION

(11) **EP 4 085 905 B1**
(45) Date of publication and mention of the grant of the patent: **21.02.2024**
(21) Application number: 21172735.9
(22) Date of filing: 07.05.2021
(51) Int. Cl.: A61K 31/047, A61K 31/728, A61P 25/00, A61P 43/00

(54) **HYALURONIC ACID FOR USE IN PREVENTING OR TREATING NEUROLOGICAL DYSFUNCTION OF NERVES OR NERVE CELLS**
HYALURONSÄURE ZUR VERWENDUNG BEI DER VORBEUGUNG ODER BEHANDLUNG NEUROLOGISCHER DYSFUNKTIONEN VON NERVEN ODER NERVENZELLEN
ACIDE HYALURONIQUE UTILISÉ POUR PRÉVENIR OU TRAITER LE DYSFONCTIONNEMENT NEUROLOGIQUE DES NERFS OU DES CELLULES NERVEUSES

(43) Date of publication of application: 09.11.2022
(73) Proprietor: TRB Chemedica AG, 85622 Feldkirchen (DE)
(72) Inventor: KALKBRENNER, Hans, 85591 Vaterstetten (DE)
(74) Representative: Maiwald GmbH

(56) References cited:
- EP-A1- 1 767 211
- WO-A1-2009/018613
- WO-A1-2019/106671
- JP-A- 2006 327 958
- JP-A- 2008 127 346
- SU YU-CHI ET AL: "The efficacy of hyaluronic acid for carpal tunnel syndrome: a randomized double-blind clinical trial", PAIN MEDICINE, BLACKWELL SCIENCE, MALDEN, US, 22 March 2021 (2021-03-22), XP009531191, ISSN: 1526-2375, DOI: 10.1093/PM/PNAB109

## Description

The present invention relates to a composition comprising hyaluronic acid for use in preventing or treating neurological dysfunction of at least one nerve or nerve cell, wherein the method is used in combination with surgical methods to treat a pinched nerve, preferably neurolysis.

Benefits of hyaluronic acid in medicine and cosmetic treatments have been known for many years.

Su Yu-Chie et al. ("The efficacy of hyaluronic acid for carpal tunnel syndrome: a randomized double-blind clinical trial", Pain Medicine, Blackwell Science, Malden, US, 22 March 2021, XP009531191) discloses a randomized double-blind clinical trial of hyaluronic acid for use in the treatment of carpal tunnel syndrome. Patients receiving the active were injected with 25 ml of a 10 % hyaluronic acid (600-1200 kDa) solution, resulting in improvement of symptoms relative to controls.

JP 2006327958 A discloses a neurological-disease-treatment agent or preventive agent, which uses hyaluronic acid as an active ingredient. Examples of neurological diseases include multiple sclerosis, neuritis, neuralgia, neuroparalysis, Parkinson's disease, Alzheimer's disease, and peripheral neuropathy.

WO 2019/106671 A1 discloses compositions comprising hyaluronic acid, a laminin polypeptide, and an antioxidant for use in treating neurogenic shock following nerve injury in a subject.

WO 2009/018613 A1 discloses a method for treating, for instance, carpal tunnel syndrome, said method comprising topical, systemic or local administration of mesenchymal precursor cells and hyaluronic acid.

EP 1767211 A1 discloses pharmaceutical compositions containing hyaluronan as an active ingredient for the treatment or prevention of inflammation or neural dysfunction, for instance peripheral neuropathy. Various forms of administration are disclosed, including administration *via* injection, topical application, and inhalation.

JP 2008127346 A discloses hyaluronan as an active ingredient for use in the treatment of neurological diseases, examples of which include neuritis, neuralgia, neuroparalysis, depression, and peripheral neuropathy.

The inventors of the present invention have surprisingly found that hyaluronic acid is suitable to improve or prevent clinical symptoms of neurological dysfunctions at nerves or nerve cells, in particular in carpal tunnel syndrome or other conditions in which a nerve is pinched or trapped.

Consequently, in a first aspect, the invention relates to a composition comprising hyaluronic acid for use in a method for preventing or treating neurological dysfunction of at least one nerve or nerve cell, wherein the method is used in combination with surgical methods to treat a pinched nerve, preferably neurolysis.

Preferably, the composition according to the invention is used in preventing or treating neurological dysfunction of at least one nerve or nerve cell in a condition that involves a pinched or compressed nerve, including without limitation carpal tunnel syndrome.

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more.

Numeric values specified without decimal places here refer to the full value specified with one decimal place, i.e. for example, 99 % means 99.0 %, unless otherwise defined.

If reference is made herein to a molecular weight, it refers to the weight average molecular weight M_{w}, if not explicitly stated otherwise. The weight average molecular weight M_{w} can be determined, e.g. by size exclusion chromatography (SEC), gel permeation chromatography (GPC) or by matrix assisted laser deionization/ionization - time of flight mass spectroscopy (MALDI-TOF-MS). If not stated otherwise, all given molecular weights are those determined by GPC. The number average molecular weight Mₙ can be determined by GPC, as described for M_{w}. The GPC determination method is well known to the person skilled in the art. In various embodiments, the polydispersity index (M_{w}/Mₙ) is in the range of from 1.0 to about 2.0

Embodiments, features, and advantages of the invention become apparent to the person skilled in the art in the following detailed description and claims. Each feature from one embodiment of the invention can be used in any other embodiment of the invention. Furthermore, the examples contained herein are intended to describe and illustrate the invention, but do not restrict it. In particular, the invention is not limited to these examples.

The scope of protection is defined by the appended claims.

The present invention relates to a composition comprising hyaluronic acid for use in a method for preventing or treating neurological dysfunction of at least one nerve or nerve cell, wherein the method is used in combination with surgical methods to treat a pinched nerve, preferably neurolysis.

Said methods may comprise topically administering the composition to the affected nerve or nerve cell. Said administration may occur in the course of a surgical intervention, such as neurolysis. "Surgical interventions" also include minimally invasive procedures such as endoscopy.

A "pinched nerve" means, in the context of the present invention, preferably a nerve that is compressed or trapped, e.g. by scarred adhesions or by other means, such as tissues that exert pressure on the nerve, said tissues including, but not being limited to, bones, muscles, cartilage or tendons.

In various embodiments, such a condition of a pinched nerve may be surgically treated by a procedure called neurolysis, with the presently claimed methods being supportive treatments that increase the efficiency of the primary treatment, i.e. the surgical treatment.

"Treatment" or "treating", as used herein, refers to reducing, alleviating or eliminating the given disease, condition, disorder or symptoms thereof. "Treating" or "treatment" does not require complete alleviation of symptoms and does not require a cure.

"Prevention" or "preventing", as used herein, refers to avoiding or reducing the severity of the given disease, condition, disorder or symptoms thereof, typically prior to its/their onset.

In the context of this invention, "neurolysis" means the intervention, in particular by surgery or minimally invasive procedures, e.g. by endoscopy, to expose a pinched nerve or to remove the pinch or incarceration.

"Neurological dysfuntions" typically mean, if not stated otherwise, any disorder of the nervous system, in particular disorders associated with pain due to a pinched nerve.

In a preferred embodiment, said at least one nerve or nerve cell treated is at least one nerve or nerve cell of the arm or hand, in particular the median nerve. Alternatively, it may be in the lower spine or the leg.

Preferably, the at least one nerve or nerve cell is at least one animal or human nerve or nerve cell. Accordingly, the uses, methods and applications described herein are carried out in a subject, said subject typically being an animal or human, preferably a human.

Hyaluronic acid, also called hyaluronan or hyaluronate, is a glycosaminoglycan. Hyaluronic acid can be present as a free acid or a pharmaceutically acceptable salt thereof, typically in form of hyaluronic acid sodium salt (or sodium hyaluronate or sodium hyaluronan). Hyaluronic acid is a naturally occurring biopolymer having biological functions from bacteria up to higher animals including humans. In animals, it is one of the chief components of the extracellular matrix. It contributes significantly to cell proliferation and migration, and may also be involved in the progression of some malignant tumors. Hyaluronan is naturally found in many tissues of the body such as skin, cartilage and the vitreous humor. It is therefore well suited to biomedical applications targeting these tissues.

In certain embodiments, the hyaluronic acid is selected from the group including, but not being limited to hyaluronic acid of dog, cat, horse, cow, pig, rooster, monkey, ape, chimpanzee, sheep, human, vegetal or microbial origin. In various embodiments, the hyaluronic acid can be derived from a natural non-mammalian source, such as (recombinant) bacterial fermentation or via chemical extraction from rooster combs.

Preferably, the hyaluronic acid may comprise or consist of natural, non-mammalian hyaluronic acid, wherein the hyaluronic acid is preferably from a bacterial fermentation source. The bacterial fermentation source is, for example, a *Streptococcus* or *Bacillus* strain.

"Natural hyaluronic acid" typically means non-chemically modified hyaluronic acid.

In various embodiments, the composition comprises hyaluronic acid, wherein the hyaluronic acid comprises or consists of non-cross-linked hyaluronic acid.

The hyaluronic acid that can be used in the present invention can be of any molecular weight M_{w}, for example from about 100 kDa to several million Da.

In preferred embodiments, the hyaluronic acid has an average molecular weight M_{w} of 100,000 to 6,000,000 g/mol, preferably 500,000 to 5,000,000 g/mol, more preferably 900,000 to 3,000,000 g/mol, in particular 1,000,000 to 2,000,000 g/mol.

In preferred embodiments, the composition comprises hyaluronic acid in a concentration of 0.1 to 10 %, preferably 0.5 to 5 %, in particular 1 % or 2 %.

Preferably, the composition comprising hyaluronic acid is a viscous solution. Typically, the solution is suitable for injection, i.e. administration by injection. The hyaluronic acid used for preparing such a composition can be, e.g. in form of fibers or powder which is dissolved or dispersed in a suitable liquid medium, typically an aqueous medium.

In various embodiments, the composition according to the invention comprises a solvent, e.g. water, and is a liquid aqueous composition. Additionally, it is possible that the composition further comprises additives such as preservative agents, dyes, buffer substances, pH-adjusting agents, growth factors, hormones or further actives.

In preferred embodiments, the composition further comprises a sugar or sugar alcohol, including without limitation mannitol and/or sorbitol. The compounds protect the hyaluronic acid from depolymerization by free radicals, thus making the hyaluronic acid more stable in the described formulations.

Preferably, the composition comprises mannitol in a concentration of 0.1 to 2 %, more preferably 0.2 to 1 %, in particular 0.5 %. In particular, the composition comprising hyaluronic acid according to the invention is used for the treatment of conditions that involve a pinched nerve, such as carpal tunnel syndrome. Said treatment may occur as an alternative to or supplement to other therapies, including surgery.

In various embodiments, the composition is administered topically around the pinched nerve(s) or nerve cell(s). The nerve may be that in an arm or hand of a subject, typically a human. Preferably, the composition is administered around the median nerve. According to the invention, said administration occurs in combination with surgical methods to treat a pinched nerve, for instance during surgery and/or by injection.

In various embodiments, the administration of the composition comprising hyaluronic acid is carried out by injection. The injection may be into the nerve sheath, i.e. the so-called epineurium. The administration may be carried out during a surgical intervention. In particular, the composition remains completely in the nerve sheath of the at least one pinched nerve after injection.

### Examples

### Example 1:

A composition comprising a 2 % fermentative, native, non-cross-linked hyaluronic acid (40 mg/2 mL with 10 mg mannitol) is used in the treatment of the carpal tunnel syndrome, whereby the hyaluronic acid composition is administered topically in an intraoperative treatment around the median nerve. The composition is provided in standard prefabricated syringes.

Compared to a 1 % fermentative, native non-cross-linked hyaluronic acid (20 mg/2mL with or without mannitol), the 2 % composition has slower flow properties that support the enclosure of the nerve with the hyaluronic acid.

The injection can be administered around the nerve track or into the epineurium. Depending on the individual anatomy, 1-2 mL are sufficient.

In such clinical applications, patients treated according to a method of the invention in addition to the surgical procedure showed better wound healing, less recurrence and faster recovery compared to patients only receiving surgical treatment.

## Claims

1. A composition comprising hyaluronic acid for use in a method for preventing or treating neurological dysfunction of at least one nerve or nerve cell, wherein the method is used in combination with surgical methods to treat a pinched nerve, preferably neurolysis.

2. The composition comprising hyaluronic acid for use according to claim 1, wherein the pinched nerve or nerve cell is a nerve or nerve cell of the arm or hand, in particular the median nerve.

3. The composition comprising hyaluronic acid for use according to any one of claims 1 to 2, wherein the hyaluronic acid comprises or consists of non-cross-linked hyaluronic acid.

4. The composition comprising hyaluronic acid for use according to any one of claims 1 to 3, wherein the hyaluronic acid comprises or consists of natural, non-mammalian hyaluronic acid.

5. The composition comprising hyaluronic acid for use according to any one of claims 1 to 4, wherein the hyaluronic acid comprises or consists of hyaluronic acid from a bacterial fermentation source.

6. The composition comprising hyaluronic acid for use according to any one of claims 1 to 5, wherein the hyaluronic acid has an average molecular weight of 100,000 to 6,000,000 g/mol, preferably 1,000,000 to 2,000,000 g/mol.

7. The composition comprising hyaluronic acid for use according to any one of claims 1 to 6, wherein the composition comprises or consists of hyaluronic acid in a concentration of 0.1 to 10 %, preferably 0.5 to 5 %, in particular 1 % or 2 %.

8. The composition comprising hyaluronic acid for use according to any one of claims 1 to 7, wherein the composition further comprises a sugar or sugar alcohol, preferably mannitol or sorbitol.

9. The composition comprising hyaluronic acid for use according to claim 8, wherein the composition comprises mannitol in a concentration of 0.1 to 2 %, preferably 0.2 to 1 %, in particular 0.5 %.

10. The composition comprising hyaluronic acid for use according to any one of claims 1 to 9, wherein the method comprises the treatment of carpal tunnel syndrome.

11. The composition comprising hyaluronic acid for use according to any one of claims 1 to 10, wherein the composition is administered topically to the at least one pinched nerve or nerve cell, in particular to the median nerve.

12. The composition comprising hyaluronic acid for use according to any one of claims 1 to 11, wherein the composition is administered by injection, preferably by injection into the epineurium.

## Patentansprüche

1. Zusammensetzung umfassend Hyaluronsäure zur Verwendung in einem Verfahren zur Vorbeugung oder Behandlung einer neurologischen Funktionsstörung mindestens eines Nervs oder einer Nervenzelle, wobei das Verfahren in Kombination mit chirurgischen Verfahren zur Behandlung eines eingeklemmten Nervs, vorzugsweise der Neurolyse, verwendet wird.

2. Die Zusammensetzung umfassend Hyaluronsäure zur Verwendung gemäß Anspruch 1, wobei der eingeklemmte Nerv oder die Nervenzelle ein Nerv oder eine Nervenzelle des Arms oder der Hand ist, insbesondere der Nervus medianus.

3. Die Zusammensetzung umfassend Hyaluronsäure zur Verwendung gemäß einem der Ansprüche 1 bis 2, wobei die Hyaluronsäure nicht vernetzte Hyaluronsäure umfasst oder daraus besteht.

4. Die Zusammensetzung umfassend Hyaluronsäure zur Verwendung gemäß einem der Ansprüche 1 bis 3, wobei die Hyaluronsäure natürliche, nicht von Säugetieren stammende Hyaluronsäure umfasst oder daraus besteht.

5. Die Zusammensetzung umfassend Hyaluronsäure zur Verwendung gemäß einem der Ansprüche 1 bis 4, wobei die Hyaluronsäure Hyaluronsäure aus einer bakteriellen Fermentationsquelle umfasst oder daraus besteht.

6. Die Zusammensetzung umfassend Hyaluronsäure zur Verwendung gemäß einem der Ansprüche 1 bis 5, wobei die Hyaluronsäure ein mittleres Molekulargewicht von 100.000 bis 6.000.000 g/mol, vorzugsweise 1.000.000 bis 2.000.000 g/mol, aufweist.

7. Die Zusammensetzung umfassend Hyaluronsäure zur Verwendung gemäß einem der Ansprüche 1 bis 6, wobei die Zusammensetzung Hyaluronsäure in einer Konzentration von 0,1 bis 10 %, vorzugsweise 0,5 bis 5 %, insbesondere 1 % oder 2 %, umfasst oder daraus besteht.

8. Die Zusammensetzung umfassend Hyaluronsäure zur Verwendung gemäß einem der Ansprüche 1 bis 7, wobei die Zusammensetzung des Weiteren einen Zucker oder Zuckeralkohol, vorzugsweise Mannitol oder Sorbitol, umfasst.

9. Die Zusammensetzung umfassend Hyaluronsäure zur Verwendung gemäß Anspruch 8, wobei die Zusammensetzung Mannitol in einer Konzentration von 0,1 bis 2 %, vorzugsweise 0,2 bis 1 %, insbesondere 0,5 %, umfasst.

10. Die Zusammensetzung umfassend Hyaluronsäure zur Verwendung gemäß einem der Ansprüche 1 bis 9, wobei das Verfahren die Behandlung des Karpaltunnelsyndroms umfasst.

11. Die Zusammensetzung umfassend Hyaluronsäure zur Verwendung gemäß einem der Ansprüche 1 bis 10, wobei die Zusammensetzung topisch auf den mindestens einen eingeklemmten Nerv oder die Nervenzelle, insbesondere auf den Nervus medianus, verabreicht wird.

12. Die Zusammensetzung umfassend Hyaluronsäure zur Verwendung gemäß einem der Ansprüche 1 bis 11, wobei die Zusammensetzung durch Injektion, vorzugsweise durch Injektion in das Epineurium, verabreicht wird.

## Revendications

1. Composition comprenant de l'acide hyaluronique pour utilisation dans un procédé de prévention ou de traitement d'un dysfonctionnement neurologique d'au moins un nerf ou une cellule nerveuse, sachant que le procédé est utilisé en combinaison avec des procédés chirurgiques pour traiter un nerf pincé, de préférence la neurolyse.

2. La composition comprenant de l'acide hyaluronique pour utilisation selon la revendication 1, sachant que le nerf pincé ou la cellule nerveuse est un nerf ou une cellule nerveuse du bras ou de la main, en particulier le nerf médian.

3. La composition comprenant de l'acide hyaluronique pour utilisation selon l'une quelconque des revendications 1 à 2, sachant que l'acide hyaluronique comprend ou est constitué par de l'acide hyaluronique non réticulé.

4. La composition comprenant de l'acide hyaluronique pour utilisation selon l'une quelconque des revendications 1 à 3, sachant que l'acide hyaluronique comprend ou est constitué par de l'acide hyaluronique naturel non mammalien.

5. La composition comprenant de l'acide hyaluronique pour utilisation selon l'une quelconque des revendications 1 à 4, sachant que l'acide hyaluronique comprend ou est constitué par de l'acide hyaluronique provenant d'une source de fermentation bactérienne.

6. La composition comprenant de l'acide hyaluronique pour utilisation selon l'une quelconque des revendications 1 à 5, sachant que l'acide hyaluronique a un poids moléculaire moyen de 100 000 à 6 000 000 g/mol, de préférence 1 000 000 à 2 000 000 g/mol.

7. La composition comprenant de l'acide hyaluronique pour utilisation selon l'une quelconque des revendications 1 à 6, sachant que la composition comprend ou est constituée par de l'acide hyaluronique dans une concentration de 0,1 à 10 %, de préférence 0,5 à 5 %, en particulier 1 % ou 2 %.

8. La composition comprenant de l'acide hyaluronique pour utilisation selon l'une quelconque des revendications 1 à 7, sachant que la composition comprend en outre un sucre ou un alcool de sucre, de préférence du mannitol ou du sorbitol.

9. La composition comprenant de l'acide hyaluronique pour utilisation selon la revendication 8, sachant que la composition comprend du mannitol dans une concentration de 0,1 à 2 %, de préférence 0,2 à 1 %, en particulier 0,5 %.

10. La composition comprenant de l'acide hyaluronique pour utilisation selon l'une quelconque des revendications 1 à 9, sachant que le procédé comprend le traitement du syndrome du canal carpien.

11. La composition comprenant de l'acide hyaluronique pour utilisation selon l'une quelconque des revendications 1 à 10, sachant que la composition est administrée topiquement à l'au moins un nerf pincé ou la cellule nerveuse, en particulier au nerf médian.

12. La composition comprenant de l'acide hyaluronique pour utilisation selon l'une quelconque des revendications 1 à 11, sachant que la composition est administrée par injection, de préférence par injection dans l'épineurium.
